# EUROPEAN PATENT APPLICATION

(11) **EP 2 248 489 A2**
(43) Date of publication of application: **10.11.2010**
(21) Application number: 10275031.2
(22) Date of filing: 31.03.2010
(51) Int. Cl.: A61F 2/46, A61B 17/00

(54) **Orthobiologics delivery tool**

(30) Priority: 01.04.2009 US 416704
(71) Applicant: S.S. White Technologies Inc., Piscataway, NJ 08854-3761 (US)
(72) Inventor: Pinal, Frank, Weehawken, NJ 07086 (US); Litwak, Alfred Anthony, Manasquan, NJ 08736 (US); Umbach, Brian, North Caldwell, NJ 07006 (US); Singh, Akhil Kumar, West New York, NJ 07093 (US)
(74) Representative: Albutt, Anthony John

(57) **Abstract**

A tool for delivering bio-compatible solid, fluid or mixed solid and fluid substances to an operating site within a patient. The tool has a tubular body or barrel with a central longitudinal through hole and a flow control valve for selectively allowing material to flow from a holding chamber within the barrel through an extender tip to a dispensing end of the tool. A plunger having a piston at one end may be inserted through the dispensing end of the barrel and drawn distally to aspirate material into the holding chamber using a syringe attached to the proximal end of the barrel, after which the valve is closed to retain the material in the chamber. The plunger, which fits into the through hole from either end of the barrel, is then removed and re-inserted into the proximal end of the barrel, the barrel valve is opened, and the plunger is pushed to move the material in the holding chamber through the extender tip and out the distal end of the barrel. An optional rod and multi-pronged blade mixing assembly may be inserted into the proximal end of the barrel to mix stored material after it is aspirated into the holding chamber and before it is dispensed.

## Description

### BACKGROUND OF THE INVENTION

The present invention relates to a tool for holding and dispensing orthobiologics, osteobiologics, and other biomaterials used in the treatment of bone and joint defects.

In orthopedic surgery it is often necessary to fill bone defects or cavities within a human or animal body to either support or enhance the biological repair of bone. For this purpose, a myriad of biomaterials are currently employed, including but not limited to calcium phosphate and calcium sulfate ceramics, collagen and collagen based materials, de-mineralized bone matrix, autologous and allologous bone and tissue, resorbable and non-resorbable polymers, composites, blood, bone marrow aspirate, platelet rich plasma, extracellular matrix (ECM), proteins, growth factors, organic molecules, tissue engineered medical products, and combinations thereof.

In structure, these biomaterials can either be porous or non-porous solids, composite solids, fluids, composite fluids, fluid & solid mixtures, hydrogels, pastes, putties, fibers, sponges, or any combination thereof. In origin, they are synthetic, natural, or organic.

In performing surgery to fill a bone void or joint defect, the technique and procedure used by an orthopedic surgeon to deliver a biomaterial to the void or defect site varies by both patient case and choice of biomaterial. While the patient case invariably influences the surgeon's choice of biomaterial to be used, it is the delivery system and procedure for delivering the biomaterial that often engenders complication and increases the risk of human error.

Too often, existing biomaterial delivery tools are either inaccurate, inefficacious, multi-staged and time-consuming, or limited in the type, shape, or form of material that can be delivered.

In the case of ceramics, such as calcium phosphate and calcium sulfate solids, a common method of delivery is to pour small solid items (available in a variety of sizes and shapes) into the bone cavity and then position those items as desired, using a manually operated instrument. With this prior art technique it is not feasible to quickly and accurately dispense a controlled amount of biomaterial to the desired location, especially since the working area for a surgeon in this type of procedure is typically only a few centimeters.

Thus, to ensure accuracy of biomaterial placement, the surgeon must exercise caution and sacrifice time. Further, this prior art technique only works when the configuration of the defect allows for pouring, the defect is superficial, or the size of the working area and proximity of other tissues do not obstruct the surgeon's view of the void or defect.

Frequently, however, ceramics and many of the other biomaterials are preloaded in syringes or other dispensers from which such preloaded materials are held in the barrel of a tubular delivery device which acts as a holding chamber, with a cap and a plunger at opposite ends of the tube closing the chamber and preventing the material from falling out. However, once the cap is removed, the biomaterial falls out, so that the existing cap and plunger arrangement does little more than allow for pouring out the biomaterial.

When the bone cavity to be filled is in a hard-to-reach location within the body, such as the femoral head (which can be greater than 8 inches from the lateral incision point, especially with an obese patient), the aforementioned device is useless, since it is not practical to remove the cap at the defect site within the body, when working at such a distance through the usual small incision (typically 2 cm.).

It is often desirable to mix solid biomaterials with blood, bone marrow aspirate, or platelet rich plasma before dispensing into the defect or void of the operative site. A problem that often arises, however, is that the dispensing tools that are designed for these applications are generally not capable of aspirating fluid directly into the material holding chamber and/or do not have mechanisms for mechanically mixing biomaterials and biological fluids within the same dispensing system.

For de-mineralized bone matrix (DBM), for instance, the DBM is usually packaged in a sterile vial or jar from which it is removed and placed in a container or tray for mixing. A biological fluid is usually then added to and mechanically mixed in that container, and the mixture is transferred into the dispensing tool and dispensed with the aid of a plunger. This multi-step procedure is unduly time consuming, results in considerable mixture waste, and requires substantial effort and caution on the part of the person handling the biomaterial, resulting in increased risk of human error.

The majority of the dispensing tools on the market are designed only to deliver a specific biomaterial, and thus are limited in their usability. DBM dispensers, for instance, are designed only to deliver DBM and DBM doused in a biological fluid. Since DBM is typically in a fibrous putty form, it can be forced into the tapered tubular compartment of the standard DBM dispenser, which is an open end hollow long plastic tube, where it will compress against the compartment walls, permitting the dispenser's dispensing end to be left open or unsealed. For ceramics, for instance, which comprise a large portion of the bone-biomaterial market, this design does not work, since the ceramic items tend to undergo brittle failure when compressed, causing material debris and bulk material to fall out of the dispensing chamber.

A syringe is usually used to dispense fluids such as hydrogels and growth factor treatments, and is unsuitable for dispensing solid ceramic-like material.

An object of the present invention is to provide a biomaterial dispensing tool that is capable of holding and effectively dispensing a variety of biomaterials and fluids, including solid biomaterials of various shapes, into hard-to-reach anatomical sites; and efficiently provide for mixing of aspirate and pre-filled material in a single step within an isolated sterile chamber.

### SUMMARY OF THE INVENTION

A tool for dispensing solid, fluid or mixed bio-compatible material has a tubular body or barrel with a longitudinal cavity including a material holding chamber. The cavity extends between a proximal loading end and a distal dispensing end of the barrel. A material flow control valve is disposed between the distal end of the chamber and the dispensing end of the barrel. An aspirating assembly is detachably secured to the proximal end of the tubular body for aspirating material into the holding chamber while preventing flow of any solid material contained in the chamber toward the proximal end of the tubular body. A removable slidable plunger arrangement is provided to seal the longitudinal cavity adjacent either end of the tubular body in order to facilitate aspiration of fluid and fluid mixtures into the holding chamber via the proximal end of the tubular body and dispensing of solid material and solid-fluid material mixtures from the distal end of the ubular body.

### IN THE DRAWING

Figure 1 is an isometric drawing of an orthobiologics delivery tool according to a preferred embodiment of the invention.

Figure 2 is an exploded isometric view of the tool shown in Figure 1.

Figure 3A is a top plan view of the tool shown in Figure 1.

Figure 3B is a front cross-sectional elevation view of the tool shown in Figure 3A, taken along the cutting plane 3B--3B.

Figure 4A is a top plan view of the tubular body or barrel 2 shown in Figure 1.

Figure 4B is a front cross-sectional view of the barrel 2 shown in Figure 4A, taken along the cutting plane 4B-4B.

Figure 5 is an isometric view of the flow regulation valve 3 shown in Figure 1.

Figure 6A is a top plan view of the cap 4 shown in Figure 1.

Figure 6B is a front cross-sectional view of the cap 4 shown in Figure 6A, taken along the cutting plane 6B--6B.

Figure 7 is a front elevation view of the syringe needle 5 shown in Figure 1.

Figure 8A is a top plan view of the plunger 6 shown in Figure 1.

Figure 8B is a top plan view of the plunger 6 shown in Figure 8A with the valve 51 unfastened.

Figure 8C is a front cross-sectional view of the plunger 6 shown in Figure 8B, taken along the cutting plane 8C--8C.

Figure 9A is a top plan view of the plunger 33.

Figure 9B is a front cross-sectional view of the plunger 33 shown in Figure 9A, taken along the cutting plane 9B--9B.

Figure 10A is a top plan view of the mixing assembly 36.

Figure 10B is a front cross-sectional view of the mixing assembly 36 shown in Figure 10A, taken along the cutting plane 10B--10B.

Figure 11 is a top plan view of the barrel shown in Figures 4A and 4B with the mixing assembly attached.

Figure 12 is a top plan view of the tool shown in Figure 1, in aspirating configuration.

Figure 13 is a top plan view of the tool as shown in Figure 12, illustrating the action of the plunger while aspirating.

Figure 14 is a top plan view of the tool as shown in Figure 11, in mixing configuration.

Figure 15 is a top plan view of the tool as shown in Figure 11, in dispensing configuration.

Figure 16 is a top plan view of the tool as shown in Figure 15, illustrating the action of the plunger while dispensing.

### DETAILED DESCRIPTION

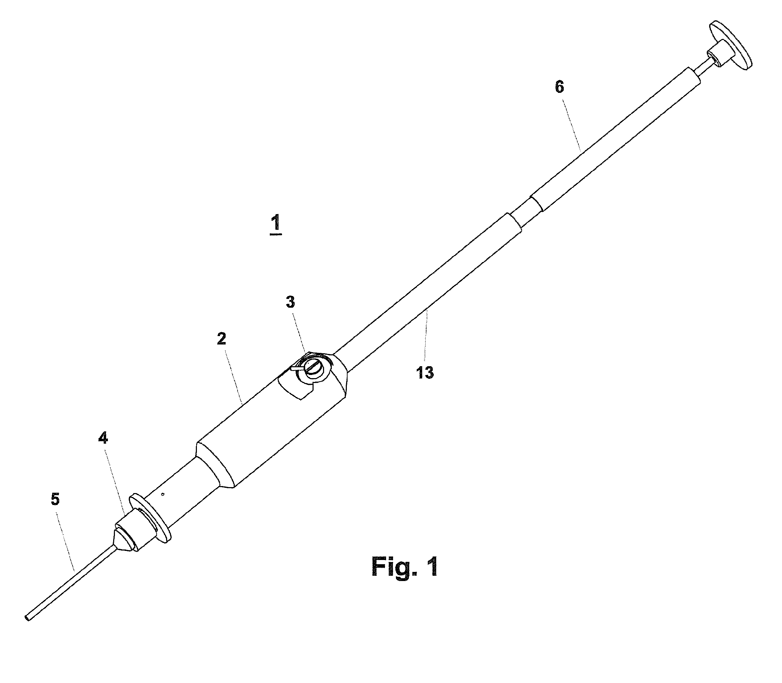

Referring to Figures 1 through 4B, the orthobiologics delivery tool 1 has a tubular body or barrel 2, flow valve 3, removable cap 4, syringe needle 5, two-shaft plunger 6, O-ring seals 7, 8 and 9, and an engagement member 10.

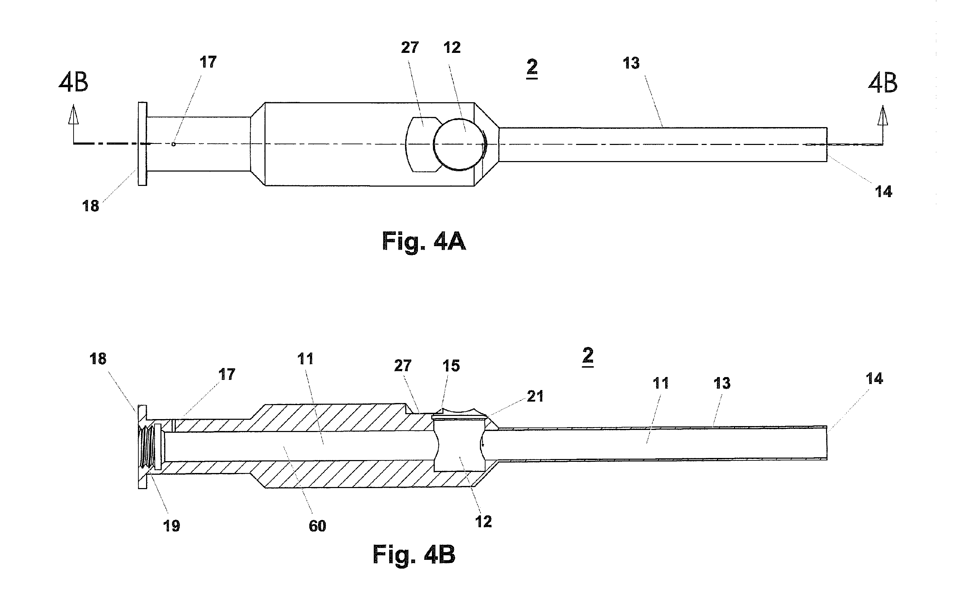

The central longitudinal cavity 11 (Figure 4B) is preferably (but not necessarily) cylindrical and runs through the entire barrel 2, and the orthobiologic or other biomaterial to be dispensed is stored in the portion of that cavity extending between the cap 4 and valve 3, which portion constitutes the material holding chamber 60.

Referring generally to Figures 4A and 4B, the barrel 2 is a hollow body whose external shape can be of any desired configuration but which is preferably generally cylindrical. The external transverse cross-sectional dimensions of the barrel 2 can vary or be constant along its length.

The cross-sectional shape of the central longitudinal cavity 11 can be round, radial, polygonal, closed polycentric curved, or closed amorphous. That cavity should preferably have transverse cross-sectional dimensions and shape that are uniform along its length, except in the area of the valve gland 12 of the valve 3.

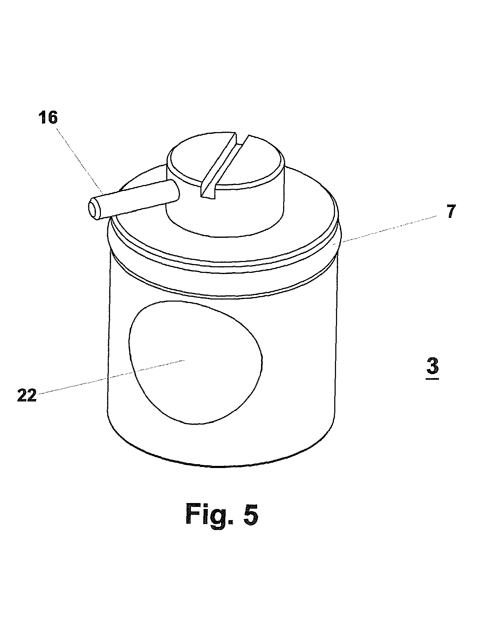

The valve gland 12 (Figure 4A) is a chamber that intersects the central cavity 11 and whose shape conforms to the external shape of the valve 3 (Figure 5).

An elongated extender tip 13 adjacent the dispensing end 14 of the barrel 2 allows for effective dispensing. The tip 13 can be of any length but is preferably at least as long as the holding chamber 60 so as to facilitate complete aspiration to the holding chamber as hereafter described, and its external shape can be round, radial, polygonal, closed polycentric curved, closed amorphous, or variable, where the shape and transverse cross-sectional dimensions along the full length of the extender tip 13, which may be integral with or attached to the tubular body or barrel 2.

A profile feature 27 on the barrel 2 comprises a material stop 15 which restricts rotational movement of the valve's stopping rod 16 and thus prevents the valve 3 (Figure 5) from further movement when the stopping structure 16 and the material stop 15 are in contact. The form, shape, and orientation of the profile feature 27 will vary with the type of valve and stopping structure that are used, where different valve types have different shapes and actuating mechanisms and can be oriented in different directions with respect to the flow of material.

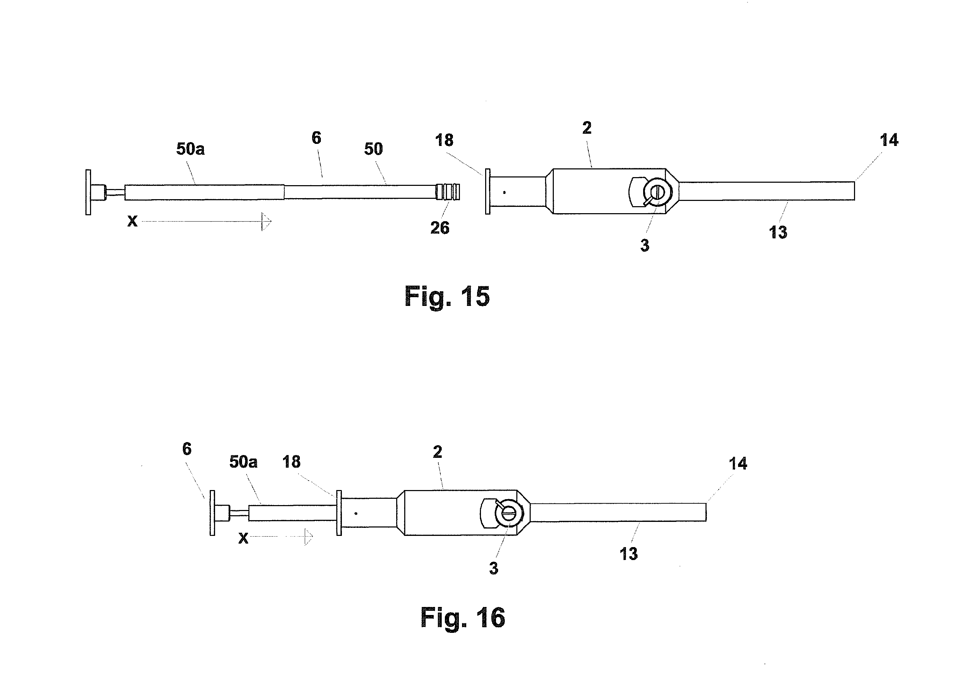

An air vent hole 17 near the end 18 of the material holding chamber 60 within the barrel 2 allows the two-shaft plunger 6 to be inserted into the central longitudinal cavity 11 at the chamber end 18 of the barrel 2 without excessive back pressure while the valve 3 is closed (see Figure 15).

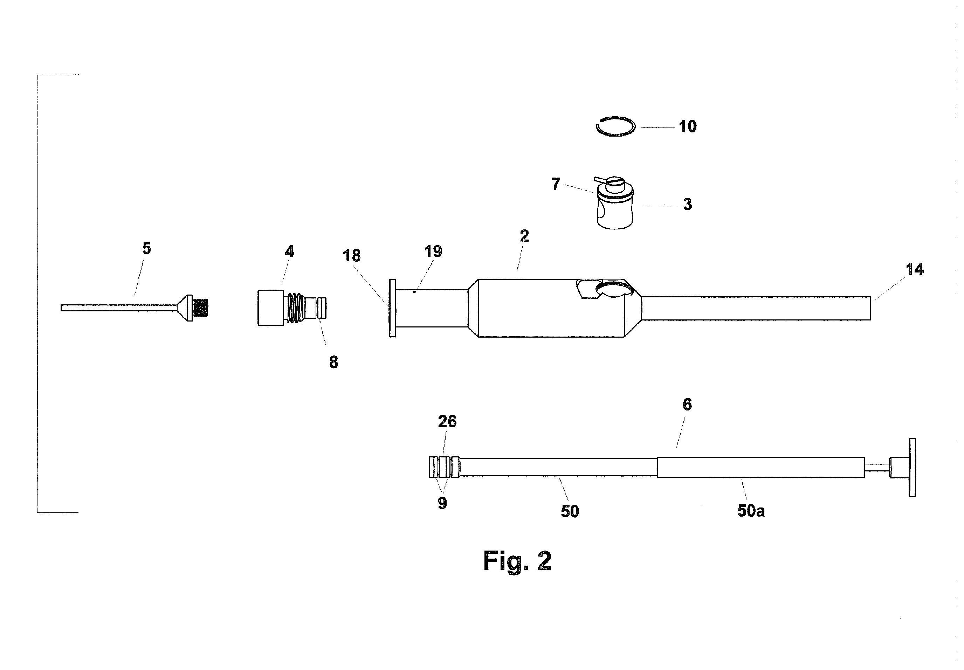

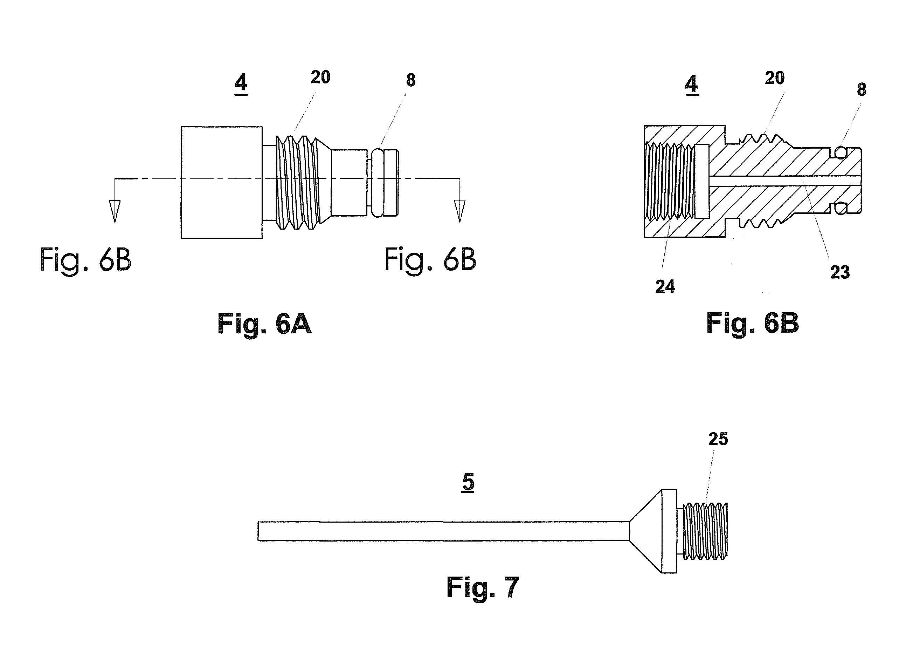

An engagement feature 19 at the chamber end 18 of the barrel 2, preferably internal threading, allows the externally threaded portion of the cap 4 (Figures 2 and 6A) to be fastened to the barrel 2 as shown in Figure 1. The engagement feature is optional, since the seal(s) 8 on the cap 4 frictionally fasten it to the central longitudinal cavity 11 on the end 18 of the barrel 2. The cap 4 may also be self-sealing, making the seal(s) 8 unnecessary. If an engagement feature 19 is used, its shape or form should engage with the engagement feature 20 on the cap 4. The form of the engagement feature 19 is that of a non-permanent fastener(s), including but not limited to: a thread, pinhole, snap ring groove, snap hook, and/or mounting boss.

Another engagement feature 21 on the barrel 2 allows the engagement member 10 (Figures 2 and 4B) to hold the valve 3 in the valve gland 12, while allowing the valve 3 to rotate between open and closed positions. The engagement feature 21 is optional as engagement may selectively be permanent or non-permanent, since the O-ring seal 7 on the valve 3 effectively fastens the valve 3 to the valve gland 12. The valve 3 may also be self-sealing, making the seal 7 unnecessary.

Referring generally to Figure 5, the valve 3 as shown is a cylinder valve, and it regulates the flow of materials and material mixtures through the material holding chamber 60 (Figure 4B) and out of the dispensing end 14 of tool 1 (Figures 2 and 15). The valve may alternatively be of any other common type, such as a ball valve, gate valve, or iris valve, etc. Its general form for this application is that of a hollow body whose central cavity 22 can have any of various orientations with respect to the central longitudinal cavity 11 of the barrel 2. The shape of the valve's central cavity 22 is adapted to selectively effect and interrupt communication between the adjacent ends of the cavity 11, and can be round, radial, polygonal, closed polycentric curved, or closed amorphous.

When the valve 3 is in an open position, meaning that material is allowed to flow, the valve's central cavity 22 aligns with the central longitudinal cavity 11 of the barrel 2, so that the valve does not obstruct material flow through the central longitudinal cavity 11. When the valve is in a closed position, material flow is blocked.

Referring generally to Figures 6A and 6B, the cannulated cap 4 has a hollow body whose purpose is both to fasten the syringe needle 5 (Figure 7) to the barrel 2 as shown in Figure 1. Due to its small cross-section, the cannula hole 23 in the cap 4 prevents the orthobiologic material from falling out of the chamber end 18 of the central longitudinal cavity 11 of the barrel 2. The external shape of the cap 4 can be round, radial, polygonal, closed polycentric curved, or closed amorphous; its external transverse cross-sectional dimensions, along its length, can be variable or constant.

The central longitudinal cavity 23 of the cap 4 can have a cross-section which is round, radial, polygonal, closed polycentric curved, or closed amorphous, and should be sufficiently small to prevent flow of the smallest unit of any solid biomaterial contained in the substance or mixture to be dispensed.

The cap 4 contains two engagement features: a permanent or non-permanent engagement feature 24 (preferably internal threading), which fastens to and is complementary in shape or form to the engagement feature 25 (preferably external threading) of the syringe needle 5, and an optional non-permanent engagement feature 20 (preferably external threading) which fastens to the engagement feature 19 (preferably internal threading) of the barrel 2 on the chamber side 18. The form of an engagement feature can be that of a fastener(s), such as but not limited to: a thread, pinhole, snap ring groove, snap hook, and/or mounting boss.

Referring to Figure 7, the syringe needle 5 has an engagement feature 25 (preferably external threading) which fastens to the engagement feature 24 (preferably internal threading) of the cap 4 (Figure 6B). The engagement feature can be that of a permanent or non-permanent fastener(s), including but not limited to a non-locking thread, pinhole, snap ring groove, snap hook, and/or mounting boss.

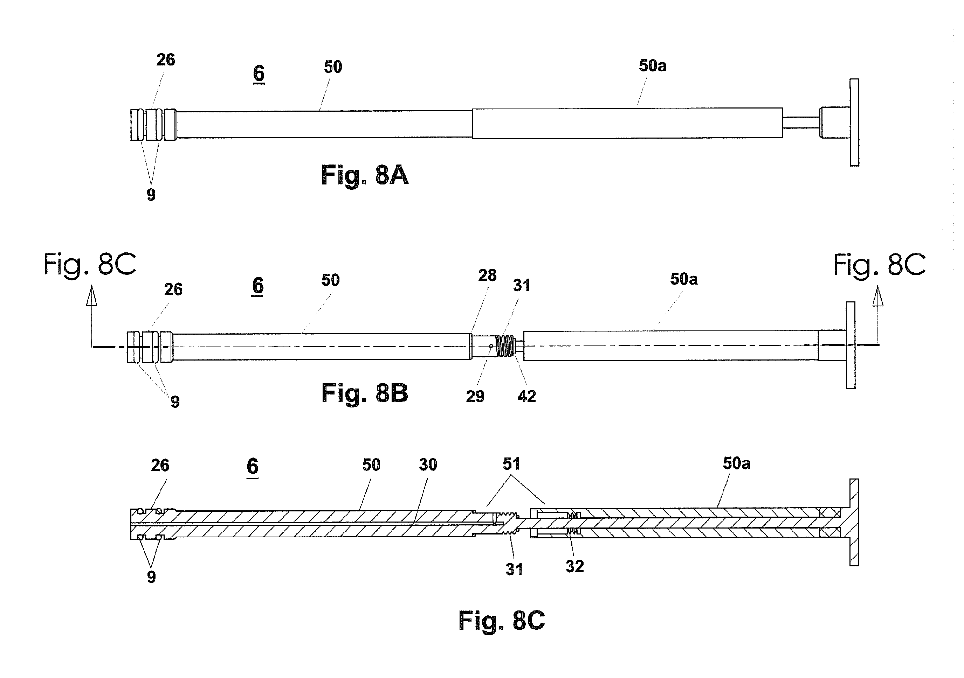

Referring generally to Figures 8A through 8C, the plunger 6 comprises a shaft 50, a second shaft 50a cooperating with the shaft 50 to provide a needle valve 51, and O-ring seals 9. The shaft 50, which may typically be 4 to 8 inches long, has a transverse cross-sectional shape at the piston 26 which conforms to that of the central longitudinal cavity 11 of the barrel 2 (Figure 4B). The seals 9 can be of any type, i.e., O-rings, grommets, molds, etc, as long as the seals are capable of maintaining a seal and/or a significant pressure difference between the internal compartments of the tool 1 (Fig 1, Figures 4A and 4B) and the atmosphere. The piston 26 may also be self-sealing, making the seals 9 unnecessary.

An axial air shaft 30 and vent hole 29 within the shaft 50 allows for air to bypass the seal created by the seals 9 at the piston 26, which is desirable when the plunger 6 is used to aspirate a large amount of fluid into the material holding chamber 60 (Figures 1, 4B and 13). The air shaft 30 may extend the entire length of the shaft 50 or less than the full length thereof, and may be oriented in a direction that is either parallel or non-parallel to the longitudinal axis of the shaft.

The orientation of the air shaft 30 and vent hole 29 are dependent upon the type of valve that is used, and the vent hole 29 is unnecessary for certain valve types.
The valve 51 as shown is a type of needle valve, but can be of any common type, i.e., a ball valve, gate valve, iris valve, cylinder valve, etc. Its general form, orientation with respect to the air holes and/or air shafts, and open-close mechanism are dependent on the type of valve used. As shown in Figure 8B, when the valve 51 is closed, the seals 28 and 42 seal the shaft 51 and prevent air from moving out of the vent hole 29 and bypassing the seal created by the seals 9 at the piston 26 of the shaft 50.

Whatever valve type is used, it should be capable of (i) preventing the passage of air through the air shaft 30 and/or vent hole 29 and (ii) maintaining the air/fluid seal created by the seals 9 at the piston 26 of the plunger 6 while the plunger 6 is lodged in the central longitudinal cavity 11 of the barrel 2 (Figure 4B). The form, shape, size, and orientation of the seals are dependant on the type of valve utilized. The seals 28 and 42 can be of any type, i.e., O-rings, grommets, molds, etc., as long as they are capable of sealing the air shaft 30 and vent hole 29. The orientation of the seals will also depend on the type of valve and seals that are used. For certain valve types, the seals may be unnecessary as the valves may be self-sealing or may have built in seals for creating or sustaining pressure differentials, or for regulating the flow of fluids or gases.

The engagement feature 31 on the shaft 50 and the engagement feature 32 on the valve 51 are optional as these are also dependent on the type of valve utilized. As shown in Figures 8B and 8C, the engagement feature 32 (internal threading) on the valve 51 engages the engagement feature 31 (external threading) on the shaft 50. These engagement features conform to each other in size and shape, and their general form is that of a non-permanent fastener(s), including but not limited to a thread, pinhole, snap ring groove, snap hook, and/or mounting boss.

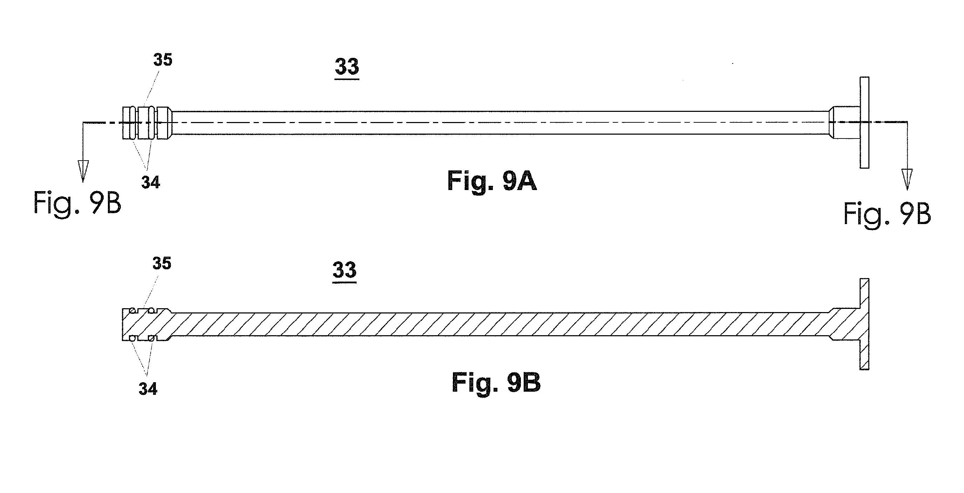

Figures 9A and 9B show a unitary plunger 33 that can be used in cases where only a small amount of aspirate is needed. The plunger 33 is a shaft typically 4 to 8 inches long with a piston 35 on its distal end and O-ring seals 34. The plunger's transverse cross-sectional shape at the piston 35 conforms to that of the central longitudinal cavity 11 of the barrel 2 (Figure 4B). The seals 34 can be of any type, i.e., O-rings, grommets, molds, etc., as long as the seals are capable of creating a seal and/or a pressure difference between the internal compartments of the tool 1 (Figures 1, 4B, and 13) and the atmosphere.

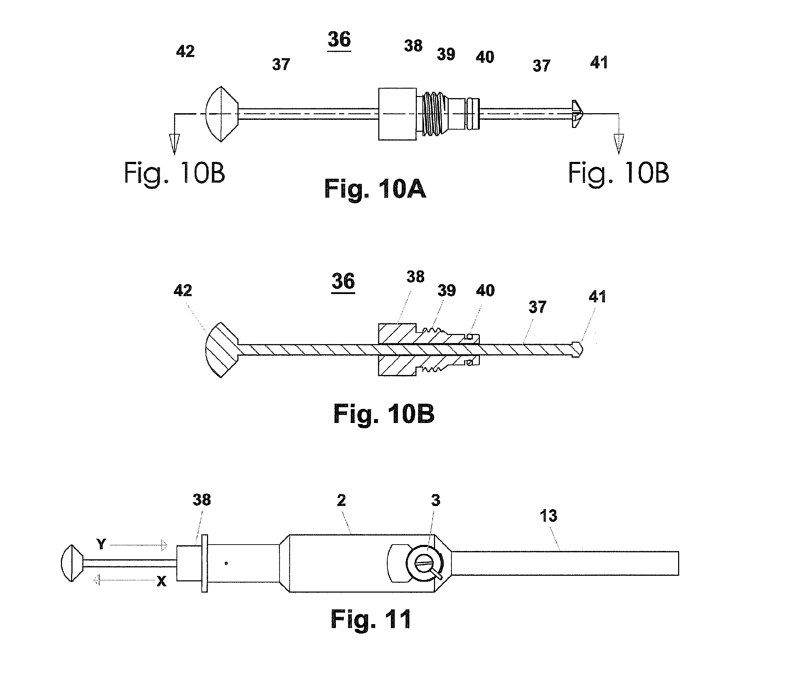

Figures 10A, 10B and 11 show the mixing assembly 36 which comprises a mixing shaft 37 and a barrel engagement member 38. The mixing shaft 37 is typically 4 to 8 inches long and has an optional grip 42 at the proximal end and a mixing tip 41 at the distal end.

The mixing tip 41 has transverse a cross-sectional shape and dimensions that allow it to move freely within the central longitudinal cavity 11 of the barrel 2 (Figure 4B). The shaft 37 can move longitudinally through the engagement member 38 in a direction that is coaxial with the longitudinal axis of the barrel 2 (Figures 4B and 15), and has an engagement feature 39 (preferably external threading) that allows the mixing assembly 36 to fasten to the engagement feature 19 (preferably internal threading) of the barrel 2. The engagement feature provides a non-permanent fastener(s), including but not limited to a non-locking thread, pinhole, snap ring groove, snap hook, and/or mounting boss. The seal 40 is optional and includes, but is not limited to O-rings, grommets, and/or molds, etc.

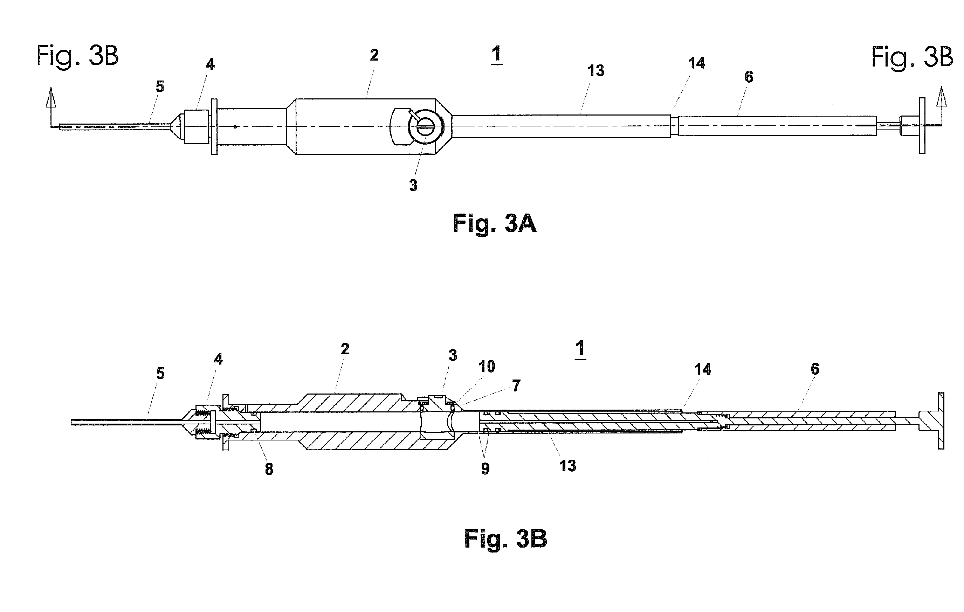

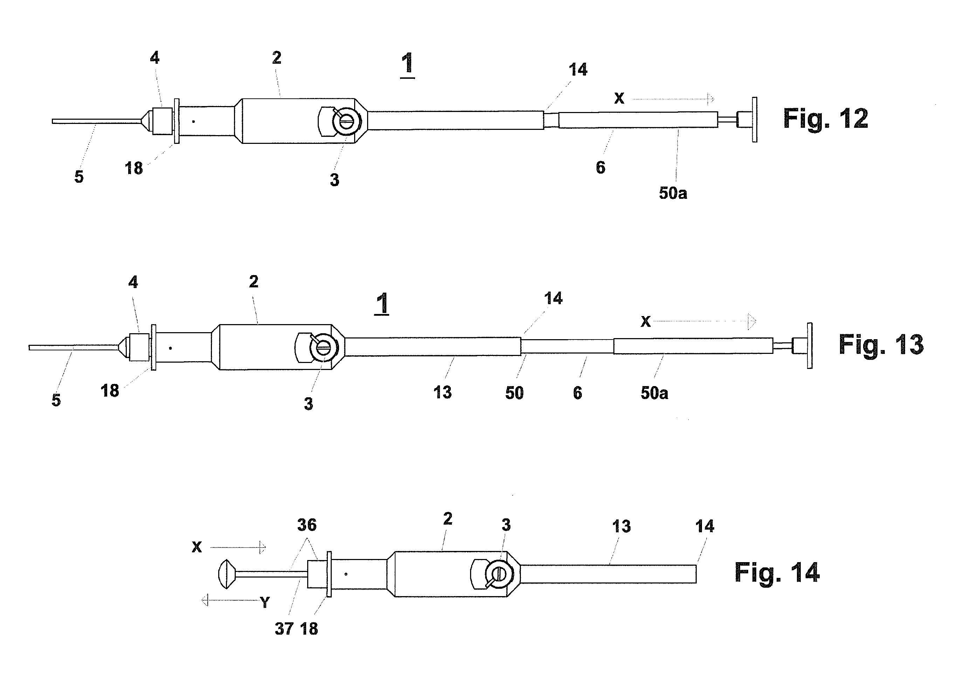

Referring to Figures 12 through 16, the plunger 6, valve 3, and mixing assembly 36 are the actuating components of the tool. When the plunger 6 is inserted into the dispensing end 14 of the tool 1 (Figures 3A, 3B, 12 and 13), the plunger 6 is positioned to aspirate (Figure 12). When the valve 3 is opened, the syringe needle 5 is submerged in a biological fluid, and the plunger 6 is pulled in a direction causing it to move away from the valve 3 (Figure 13). This action causes fluid to aspirate through the syringe needle 5 and into the material holding chamber 60 of the barrel 2, which is where the orthobiologic material to be dispensed is stored.

The valve 3 is then closed, and the cap 4 and syringe needle 5 are then dissembled and removed from the tool 1. The mixing assembly 36 can then be substituted for the cap 4, and the mixing shaft 37 can be used to mix the biomaterial and biological fluid, if desired (Figure 14), after which the mixing assembly 36 is removed from the tool.

The plunger 6 (or a different plunger) is then inserted into the chamber end 18 of the barrel 2 (Figure 15), where its piston 26 directly contacts the orthobiologic material and fluid. The dispenser end 14 of the barrel 2 is placed in the desired location for delivery of the orthobiologic material. The valve 3 is opened, and the plunger 6 is then pushed with a motion that causes it to move toward the dispensing end 14 of the barrel 2 until all material is expelled from the dispensing end 14 (Figure 16).

## Claims

1. A tool for dispensing solid, fluid or mixed biocompatible material, comprising:
a tubular body having a longitudinal cavity including a material holding chamber, said cavity extending between a proximal loading end and a distal dispensing end of said body,
said tubular body including a valve between said material holding chamber and said dispensing end for controlling the flow of aspirated material into said chamber and the flow of material to be dispensed from said chamber through said dispensing end;
a cannulated cap and syringe needle assembly removably secured adjacent the proximal end of the tubular body for aspirating material into said chamber when said valve is open, the cannula of said assembly communicating with the longitudinal cavity of said tubular body,
said cannula having a sufficiently small cross-section to prevent flow of solid material contained in said chamber through the cap and syringe needle assembly; and
a plunger having a shaft adapted to extend into said longitudinal cavity from either the proximal or the distal end thereof,
said plunger having a piston adjacent one end thereof for closely engaging the adjacent interior wall of the longitudinal cavity, so that when the valve is open the piston may, after insertion into the cavity from the distal end thereof, be moved distally to cause material to be aspirated into the proximal end of the tubular body for storage in the holding chamber, whereby said stored material may be retained in the holding chamber when the valve is closed, and when the valve is opened thereafter, the stored material may be moved to the dispensing end of the tubular body and dispensed therefrom by a piston end of a plunger shaft inserted into the proximal end of the cavity.

2. The tool according to Claim 1, further comprising a removable mixing assembly comprising a rotatable rod with a mixing element at a distal end thereof, said rod and mixing element being adapted for insertion into the longitudinal cavity so that the mixing element extends into said material holding chamber.

3. The tool according to Claim 2, further comprising means for detachably securing the mixing assembly to the tubular body adjacent the proximal end thereof.

4. The tool according to Claim 1, wherein the tubular body has an elongated extender tip adjacent the distal end thereof, said longitudinal cavity extending through said extender tip.

5. The tool according to Claim 4, wherein said extender tip has a length at least equal to the length of the material holding chamber.

6. The tool according to Claim 4, wherein the portion of said longitudinal cavity within said extender tip has a cross-section at least as great as that of the portion of said cavity constituting said material holding chamber.

7. The tool according to Claim 1, wherein the piston of said plunger is adapted to seal the longitudinal cavity adjacent either end of the tubular body, to facilitate aspiration of fluid and fluid mixtures into the holding chamber via the proximal end of the tubular body and dispensing of solid material and material mixtures from the distal end of said body.

8. The tool according to Claim 7, wherein said tubular body has an elongated extender tip adjacent the distal end thereof, said longitudinal cavity extending through said extender tip, so that when said valve is open, material in said holding chamber may be caused to flow through said extender tip and be dispensed from said distal end of said tubular body.

9. The tool according to Claim 9, wherein the plunger includes controllable venting means to selectively allow air to bypass the seal created by the piston while the piston is lodged in the longitudinal cavity of the tubular body.

10. A tool for dispensing solid, fluid or mixed biocompatible material, comprising:
a tubular body having a longitudinal cavity including a material holding chamber, said cavity extending between a proximal loading end and a distal dispensing end of said body;
a material flow control valve disposed between the distal end of said chamber and the dispensing end of said body;
aspirating means detachably secured to the proximal end of the tubular body for aspirating material into said chamber while preventing flow of solid material contained in said chamber toward the proximal end of said tubular body; and
plunger means adapted to seal the longitudinal cavity adjacent either end of the tubular body, to facilitate aspiration of fluid and fluid mixtures into the holding chamber via the proximal end of the tubular body and dispensing of solid material and material mixtures from the distal end of said body.

11. A tool for dispensing solid, fluid or mixed biocompatible material, comprising:
a tubular body having a longitudinal cavity including a material holding chamber, said cavity extending between a proximal loading end and a distal dispensing end of said body,
said tubular body including a valve between said material holding chamber and said dispensing end for controlling the flow of aspirated material into said chamber and the flow of material to be dispensed from said chamber through said dispensing end,
said tubular body having an elongated extender tip adjacent the distal end thereof, said longitudinal cavity extending through said extender tip;
a cannulated cap and syringe needle assembly removably secured adjacent the proximal end of the tubular body for aspirating material into said chamber when said valve is open, the cannula of said assembly communicating with the longitudinal cavity of said tubular body,
said cannula having a sufficiently small cross-section to prevent flow of solid material contained in said chamber through the cap and syringe needle assembly; and
a plunger having a shaft adapted to extend into said longitudinal cavity from either the proximal or the distal end thereof,
said plunger having a piston adjacent one end thereof for closely engaging and effectively sealing the adjacent interior wall of the longitudinal cavity, so that when the valve is open the piston may, after insertion into the cavity from the distal end thereof, be moved distally to cause material to be aspirated into the proximal end of the tubular body for storage in the holding chamber, whereby said stored material may be retained in the holding chamber when the valve is closed, and when the valve is opened thereafter, the stored material may be moved to the dispensing end of the tubular body and dispensed therefrom by a piston end of a plunger shaft inserted into the proximal end of the cavity,
said plunger including controllable venting means to selectively allow air to bypass the seal created by the piston when the piston is lodged in the longitudinal cavity within the extender tip of the tubular body.
